# EUROPEAN PATENT APPLICATION

(11) **EP 3 112 470 A1**
(43) Date of publication of application: **04.01.2017**
(21) Application number: 15306066.0
(22) Date of filing: 01.07.2015
(51) Int. Cl.: C12P 1/04, C12R 1/00

(54) **METHOD FOR PRODUCING ANTIMICROBIAL AGENTS**

(71) Applicant: Deinobiotics, 34790 Grabels (FR)
(72) Inventor: DHULSTER Pascal, 59000 Lille (FR); LE BELLER Dominique, 60880 Jaux (FR); LEONETTI Jean-Paul, 34790 Grabels (FR); MANDAVID Hugues, 59260 Hellemes-Lille (FR); LEVASSEUR Prémavathy, 95880 Enghien Les Bains (FR); VALENDUC Marjorie, 59320 Emmerin (FR)
(74) Representative: Hirsch & Associés

(57) **Abstract**

The present invention relates to a method for producing one or several compounds having antimicrobial activity, comprising cultivating microorganism from the genus *Microbacterium* and collecting, from said culture, a compound having antimicrobial activity produced by said microorganism.

## Description

### BACKGROUND OF THE INVENTION

Antimicrobial resistance, which entails the microorganisms ability to find ways aimed at circumventing the actions of the drugs used to cure the infections caused by such microorganisms, is held as a current public health issue not only because of the growing trend of resistant bacteria, but also due to the lack of new antibiotics.

Thus, there is a growing demand of antibiotics not only due to the resistance issue, but also to the extended life expectancy of the population.

For example, multi-drug resistant Gram-positive bacteria (MDRGP) still continue to pose challenges to the scientific community, which involve *Staphylococcus aureus,* whose first penicillin-resistant strains emerged more than fifty years ago. Also, the multiple-drug resistant Gram-negative bacteria (MDRGN) have turned into an issue of concern, particularly, the *E. coli*-resistant strains.

Therefore, the search for new chemical entities with antimicrobial properties and structures differing from those found in conventional antibiotics is viewed as a pressing need to develop new ways to curb these resistant infections. The applicant has found that *Microbacterium* is particularly useful to produce novel compounds having antimicrobial activity. All *Microbacterium* strains described in the literature so far have been isolated from environmental sources. Initially, these yellow- or orange-pigmented, fermentative gram-positive rods (GPRs) were identified as CDC coryneform group A-4 and A-5 bacteria (12), but further investigations revealed that they belong to the genus *Microbacterium* (Primary Identification of Microbacterium spp. Encountered in Clinical Specimens as CDC Coryneform Group A-4 and A-5 Bacteria, Guido FUNKE, JOURNAL OF CLINICAL MICROBIOLOGY, Jan. 1995, p. 188-192).

### BRIEF SUMMARY OF THE INVENTION

The present invention relates to a method for producing one or several compounds having antimicrobial activity, comprising cultivating microorganism from the genus *Microbacterium* and collecting, from said culture, a compound having antimicrobial activity produced by said microorganism. It has been shown that the genome of *Microbacterium* codes for enzymatic pathways producing biologically active secondary metabolites. The present invention provides a method for producing compounds having antimicrobial activity isolated from a microorganism of the genus *Microbacterium.*

### SUMMARY OF THE INVENTION

It is a primary object of the present invention to provide a method for efficiently and effectively producing such compounds having antimicrobial activity. It is a further object of the present invention to show that microorganisms of the genus *Microbacterium* are capable of efficiently and effectively producing compounds having antimicrobial activity. An object of the invention is the use of secondary metabolites produced by the genus *Microbacterium* as compounds having antimicrobial activity

In accordance with the teachings of the present invention, identified and utilized as disclosed herein are microorganism strains of the genus *Microbacterium* which are useful for the production of compounds having antimicrobial activity.

In further accordance with the teachings of the present invention, disclosed herein is a method for the production of compounds having antimicrobial activity. This method is comprised of culturing a strain of the genus *Microbacterium,* whereby compounds having antimicrobial activity are produced thereby.

Preferably, the method of the present invention comprises culturing one of the following strains of the genus *Microbacterium: Microbacterium imperiale, Microbacterium lacticum, Microbacterium ammoniaphilum, Microbacterium arborescens, Microbacterium laevaniformans* and *Microbacterium liquefaciens* / *maritypicum* / *oxydans.*

Most preferably, the method of the present invention comprises culturing the strain*Microbacterium arborescens*, and more particularly the strain*Microbacterium arborescens CIP 55.81T.*

In another related aspect of the present invention, disclosed herein is a method for the production of compounds having antimicrobial activity produced by a strain of the genus *Microbacterium.* This method includes the steps of culturing a strain of the genus *Microbacterium,* whereby compounds having antimicrobial activity are produced and recovered.

### DESCRIPTION OF PREFERRED EMBODIMENTS

The microorganisms of the genus *Microbacterium* can be cultured in any manner which permits the growth thereof with the concurrent production of compounds having antimicrobial activity, as will be readily understood by one skilled in the art. In this regard, it is contemplated herein that such cultivation will be done in culture medium which also contains sources of carbon (such as glucose, starch, glycerol, sucrose and molasses), nitrogen (such as peptone, hydrolyzed derivatives of casein, meat or soja, ammonium or sodium nitrates, ammonium sulphate or phosphate, corn steep liquor, malt extract, etc.) and, if desired, mineral salts. The pH of the aqueous culture medium is controled by buffers such as MOPS (3- (N-morpholino)propansulfonic acid), phosphoric acids salts or TRIS buffer or by continuous controled addition of sodium, potassium or ammonium hydroxide or an inorganic acid, such as hydrochloric or sulfuric acid. The pH is maintained between about 5 to about 10 (and, preferably from about 7 to about 9).

The temperature of such cultivation can be carried out at a temperature range of from about 20°C to about 40°C and preferably, from about 30° C. to about 35°C. for a period of from about 10 to about 144 hours, more preferably from about 72 to 96 hours depending upon the particular strain cultivated. The pH of the culture media may be from about pH 5 to about pH 10 with preferably a pH of about 7 to about 9.

The microorganisms of the genus *Microbacterium* may then be recovered at the end of the cultivation. Such recovery may be carried out by any suitable means therefor which are well known to those skilled in the art. As disclosed herein, such recovery is achieved by centrifugation of the culture broth so as to form a pellet containing the cells, followed by decanting of the supernatant or otherwise recovering the cells. Although the microorganism may be used in a free form of bacterial cell, it is possible to immobilize the bacterial cell. In such an event, the bacterial cells may be immobilized while in (before the removal thereof from) the whole fermentation. After such immobilization, the fermentation broth may be easily removed therefrom by any method well known to those skilled in the art, such as by simple decanting.

Immobilization of the bacterial cells may be done by any conventional method well-known to those skilled in the art, suitable to permit the use according to the method of the present invention. Such methods include being immobilized in alginate or carrageenan gel or a high polymer film.

When the culture is terminated, the supernatant and the pellet are separated. The extraction of the compounds having antimicrobial activity produced by a strain of the genus *Microbacterium* is carried out on the pellet or the supernatant or on both with a polar solvent or a non-polar solvent, or a mixture thereof optionally followed by a purification process which enriches the compounds having antimicrobial activity from the polar solvent or the non-polar solvent.

In one embodiment of this aspect of the invention, the polar solvent for extraction or purification independently include (alone or mixtures), but is not limited to C1-C4 alcohol (e.g. methanol, ethanol, isopropanol, butanol), dimethyl sulfoxide, tetrahydrofuran, acetone, acetonitrile, or mixtures thereof.

In one embodiment of this aspect of the invention, the non-polar solvent for extraction or purification independently include (alone or mixtures), but is not limited to hexanes, heptanes, ethyl ether, ethyl acetate, petroleum ether, dichloromethane, chloroform, toluene, methyl tert-butyl ether, methyl isobutyl ketone, or mixtures thereof. Mixtures of polar and non-polar solvents can also be used, for example mixtures of chlorinated solvents and alcohols like dichloromethane/methanol (DCM).

The first embodiment of the present invention consists in identifying and providing microorganism of the genus *Microbacterium* producing compounds having antimicrobial activity.

The second embodiment of the present invention consists in providing a production process of compounds having antimicrobial activity which comprises culturing a strain of the genus *Microbacterium* on a nutrient medium to accumulate the said compounds having antimicrobial activity in the nutrient medium and in the bacteria

*Microbacterium,* preferably *Microbacterium arborescens* is an example which can be most effectively used in the present invention. The mycological characteristics of this strain are as follows.

### Various Physiological Properties

Optimum growth condition: The most suitable growth condition of this strain is pH 5 to 9, temperature 20 to 35° C.

The *Microbacterium arborescens* strain is cultured in or on a suitable nutrient medium at first for the production of compounds having antimicrobial activity. In the culture of such a strain of the present invention, an ordinary culture method is applied generally. As for the culture media, nutrient media containing a carbon source which the microorganism can assimilate, a nitrogen source which the microorganism can digest and further inorganic salts as needed are used appropriately.

Glucose, sucrose, cane sugar, molasses, starch, dextrin, cellulose, glycerin, organic acids, peptone may be used singly or in combination as the assimilable carbon source mentioned above. Organic nitrogen source such as peptone, meat extract, yeast extract, dried yeast, soybean meal, corn steep liquor, cottonseed meal, casein, soy protein hydrolysate, amino acids and urea, inorganic nitrogen compounds such as nitrates and ammonium salts may be used singly or in combination as the digestible nitrogen source.

In addition, inorganic salts such as sodium salts, potassium salts, calcium salts, magnesium salts and phosphates, heavy metal salts may be added as needed. Furthermore, micronutrients, growth promoters and precursors which promote the growth of the microorganism of the present invention and/or the production of compounds having antimicrobial activity substances may be added as needed, properly in the culture medium.

It is usually preferable to perform culture under aerobic conditions such as shaking culture or aeration stirring culture. Industrially, submerged aeration culture is preferable. The pH of the culture medium is preferably around neutrality. The culture temperature may be in the range of 20 to 37° C., but the temperature is ordinarily maintained to the range of 24°C to 30°C, preferably around 30° C. As for the culturing time, compounds having antimicrobial activity are ordinarily produced and accumulated when culturing is performed for 3 to 6 days and therefore, the culturing may be preferably finished when the accumulated compounds having antimicrobial activity reach the maximum level.

Needless to say, these culture conditions such as the culture composition, pH of the culture medium, culture temperature, stirring rate and aeration rate may appropriately be adjusted and/or selected so that desirable results may be obtained depending on the kind of the strain to use and/or the external conditions. When foaming occurs in liquid culturing, an antifoaming agent such as silicone oil, vegetable oil and a surfactant may be used appropriately.

In order to take out the compounds having antimicrobial activity from the culture, the culture filtrate or the pellet are extracted with organics solvents such as acetone, ethyl acetate, butyl acetate, butanol, dichloromethane, methanol and chloroform or a mixture of these solvents, and the extract is concentrated under reduced pressure to obtain crude compounds having antimicrobial activity. The crude compounds having antimicrobial activity can be further subjected to known methods usually used for purification as column chromatography using carriers such as silica gel or reversed phase to separate and purify the compounds having antimicrobial activity.

### EXAMPLE

In the following, the present invention is specifically described by way of examples but the present invention is not limited to only these.

### Preculture

100 ml of a partial YPG medium containing peptone : 10 g/L, yeast extract 5 g/L, was autoclaved at 121°C for 20 minutes. After cooling down, the medium was completed by sterile MOPS solution (final concentration: 150 mM) and sterile glucose solution (final concentration 1 g/L). The pH was adjusted to pH 7.2 with a sterile solution of KOH or HCl. The medium was inoculated with a colony of the primary *Microbacterium arborescens* strain CIP 55.81T deposited in the Collection de l'Institut Pasteur (CIP) at the Biological Resource Center of Institut Pasteur and incubated at 30°C for 24 h with stirring at 160 rpm. Optical Density (OD) at 600 nm was then measured by a spectrophotometer until the *Microbacterium arborescens* strain was at the beginning /middle of its exponential growth phase (1 <OD at 600 nm <3). The purity of the preculture was monitored by seeding on YPG agar. The plates were incubated at 30°C for 48 h.

### Cultures in Erlenmeyer flasks

A 5000 ml flask, containing as a final volume 1000 ml sterile YPG medium was inoculated with the 100 ml of preculture and incubated at 30°C for 96 hours with stirring at 160 rpm. Initial OD at 600 nm ranged between 0.1 and 0.3. Purity of fermentation was monitored at the end of 96 hours by seeding a YPG agar. The plates were incubated at 30°C for 48 h. The culture was centrifuged at 10,000 g for 45 min at 25°C. The supernatant and the pellet were kept at 4°C

### Extraction of the compounds having antimicrobial activity (antimicrobial agents)

Extraction of the compounds having antimicrobial activity from the supernatant was carried out by extraction with dichloromethane /methanol (DCM) in a 80: 20 ratio. The operation was carried out 5 times. The solvent was evaporated under reduced pressure to yield the fraction DCM.

### Antimicrobial activity of the antimicrobial compound according to the invention:

The measures of activities were conducted on DCM extract, following the protocol recommended by the Clinical and Laboratory Standards Institute (CLSI) - Clinical and Laboratory Standards Institute (CLSI, formerly NCCLS): Dilution Antimicrobial Susceptibility Methods for Tests for Bacteria That Grow Aerobically; Approved Standard - Seventh Edition (2006). Clinical and Laboratory Standards Institute Document M7-A7.

Determination of minimum inhibitory concentrations (MICs) on DCM sample in DMSO

| **STRAIN** | **MIC** - **µg**/**mL** |
|---|---|
| *S. aureus -* ATCC 13709 (sensitive) | 20 |
| *S. aureus* MRSA - ATCC 1683 (Methicillin resistant) | 40 |

### Improvement of the extraction

The extraction was conducted as described above, but the evaporation was stopped when about 50 ml of solvent remained. After one hour, a precipitate was obtained and the remaining solvent removed yielding the precipitate (PREC)

### Determination of minimum inhibitory concentrations (MICs) on PREC in DMSO

| **STRAIN** | **MIC - µg**/**mL** |
|---|---|
| *S. aureus -* ATCC 13709 (sensitive) | 5 |
| *S. aureus* MRSA - ATCC 1683 (Methicillin resistant) | 10 |

## Claims

1. A method for producing one or several compounds having antimicrobial activity, comprising cultivating a microorganism from the genus *Microbacterium* and collecting, from said culture, a compound having antimicrobial activity produced by said microorganism.

2. The method according to claim 1, **characterized in that** the microorganism is selected from the group consisting of *Microbacterium imperiale, Microbacterium lacticum, Microbacterium arborescens, Microbacterium liquefaciens* / *maritypicum* / *oxydans* and *Microbacterium laevaniformans.*

3. The method according to claim 1 or 2, **characterized in that** the microorganism is *Microbacterium arborescens, more particularly Microbacterium arborescens CIP 55.81T.*

4. The method according to any of claims 1 to 3, comprising culturing the microorganism in a nutrient medium comprising a source of carbon, a source of nitrogen and a yeast extract.

5. The method according to any of claims 1 to 4, comprising culturing the microorganism at a temperature range of from about 20°C to about 40°C.

6. The method according to any of claims 1 to 5, comprising culturing the microorganism at a pH of from about 6 to about 8.

7. The method according to any of claims 1 to 6, comprising culturing the microorganism for a period of from about 10 to about 144 hours.

8. The method according to any of claims 1 to 7, comprising extracting the compound having antimicrobial activity by a liquid-liquid extraction in contact with a polar or a non-polar solvent or mixtures thereof.

9. A compound having antimicrobial activity obtainable by a method of anyone of claims 1 to 9.

10. Use of a bacterium of the genus *Microbacterium* for the production of a compound having antimicrobial activity.

11. Use according to claim 10, **characterized in that** the bacterium is selected from the group consisting of Microbacterium imperiale, Microbacterium lacticum, Microbacterium arborescens, Microbacterium liquefaciens / maritypicum / oxydans and Microbacterium laevaniformans.

12. Use according to claim 10 or 11, **characterized in that** the microorganism is *Microbacterium arborescens.*

13. Use according to claim 10 or 11, **characterized in that** the microorganism is *Microbacterium* sp.
